(19) Europäisches Patentamt
European Patent Office
Office européen des brevets

(11) EP 0 568 594 B1

(12) EUROPEAN PATENT SPECIFICATION

(45) Date of publication and mention
of the grant of the patent:
**17.04.1996 Bulletin 1996/16**

(51) Int Cl.$^6$: **C07K 14/62**, A61K 38/28
// C07K99:26

(21) Application number: **92903864.4**

(22) Date of filing: **22.01.1992**

(86) International application number:
**PCT/DK92/00019**

(87) International publication number:
**WO 92/12999 (06.08.1992 Gazette 1992/21)**

(54) **NOVEL INSULIN ANALOGS suited for transdermal application**

Neue Insulinanaloge geeignet für die transdermale Anwendung

NOUVEAUX ANALOGUES D'INSULINE pour l'administration transdermale

(84) Designated Contracting States:
**AT BE CH DE DK ES FR GB GR IT LI LU NL SE**

(30) Priority: **22.01.1991 DK 101/91**

(43) Date of publication of application:
**10.11.1993 Bulletin 1993/45**

(73) Proprietor: **NOVO NORDISK A/S**
**DK-2880 Bagsvaerd (DK)**

(72) Inventor: **BRANGE, Jens, Jorgen, Veilgaard**
**DK-2930 Klampenborg (DK)**

(74) Representative: **Jorgensen, Dan et al**
**Novo Nordisk A/S,**
**Patent Department,**
**Novo Allé**
**DK-2880 Bagsvaerd (DK)**

(56) References cited:
**EP-A- 0 214 826       WO-A-89/10937**
**WO-A-90/07522**

• **NATURE, vol. 333, no. 6174, 16 June 1988; J.**
**BRANGE et al., pp. 679-682**

## Description

### FIELD OF THE INVENTION

The present invention relates to novel insulin analogs for transdermal administration by iontophoresis, to compositions containing the novel insulin analogs and to the use of the insulin analogs for the treatment of diabetes by transdermal administration by iontophoresis.

### BACKGROUND OF THE INVENTION

The transdermal administration of drugs in order to obtain systemic effects has gained increasing recognition in recent years and this administration method is increasingly being used for delivery of low molecular weight drugs. The conventional transdermal delivery by passive diffusional transport of the drug across the skin barrier is useful only for delivery of drugs which are relatively small in molecular size and lipophilic (hydrophobic) in nature. The anti-sea sickness patch and the nitroglycerin patch to which this mode of administration has been applied are both low molecular weight drugs which are lipophilic (hydrophobic) in character.

However, passive transdermal delivery is not well suited to, and may not even be possible for drugs which are low in skin permeability. Unfortunately, such drugs include pharmacologically active hydrophilic peptides or proteins.

In order to obviate the limitations on passive forms of transdermal delivery, the art has considered iontophoresis for systemic delivery of ionizied hydrophilic drugs. Iontophoresis may be described as being the transfer of solutes through a biologic membrane under the influence of an electrical current.

Transport through the skin by iontophoresis is believed to occur primarily through aqueous pores in the skin, e.g. sweat ducts. Since the net polarity of the pores in human skin is negative at neutral pH, delivery of positively charged molecules therethrough can be facilitated by application of an electrical current.

The efforts made by the art, heretofore, to administer insulin through iontophoresis have been without great success. See Stephen, R. L. et al. "Potential novel methods for insulin administration: I. Iontophoresis", Biomed. Biochim. Acta **43** (1984) 553 - 558. Stephen et al. attributed their failure to force insulin across the skin barrier in pharmacologically adequate amounts to the relatively large size of the hexamer form of the insulin molecules (in solution). Stephen et al. postulated that a more strongly ionized predominantly monomeric form of insulin might sucessfully be administered transdermally by iontophoresis.

Subsequent to the work of Stephen et al. other investigators have reported achieving a more sucessful transdermal transport of human insulin by iontophoresis, see Meyer et al., "Transdermal Delivery of Human Insulin to Albino Rabbits Using Electrical Current", The American Journal of Medical Sciences, **297** (1989) 321 - 325 and Ovais Siddiqui et al., "Facilitated Transdermal of Insulin", Journal of Pharmaceutical sciences, **76** (1987) 341 - 345.

In the treatment of diabetes, parenteral administration of a predetermined quantity of insulin is presently required in order to maintain a therapeutically effective level of insulin in the blood European Application No. EP 214 826 discloses insulin analogues being particularly rapidly absorbed after injection. This effect is a result of the fact that by means of certain substitutions in particular in the B8-12 region and in the B26-B28 positions by preferably Asp or Glu suppression of the association tendency is obtained. Due to the discomfort of the several daily (sub-cutaneous) injections of insulin that typically are administered, much effort has been expended by the art over the years to find an administration route for insulin, other than through sub-cutanous injection. Transdermal iontophoretic delivery offers the potential for being a superior route for administration of insulin. However, molecules of the usual insulin compound administered, e.g., the porcine insulin or human insulin, that heretofore has been conventional in the pharmaceutical compositions, is present in solution in such compositions in an associated form, notably as dimers and hexamers, the latter being the predominant association species.

The insulin hexamer has the shape of a slightly flattened sphere. The diameter of the insulin hexamer is approximately 50Å, which diameter is believed to be too large for transdermal delivery (through the pores in the skin) in sufficient quantities to generate the therapeutically needed blood levels of insulin.

It is the purpose of the present invention to provide novel human insulin analogs with a smaller diameter which can be driven across the skin barrier through iontophoresis in therapeutically effective amounts.

### SUMMARY OF THE INVENTION

Briefly stated, the present invention relates to a method for transdermally administering an insulin analog with a negative charge at neutral pH and with a reduced tendency to association. The method comprises placing an aqueous medium of the human insulin analog in contact with human skin then applying direct current to said insulin analog medium and therethrough to the skin in contact with the medium. Pharmacologically effective quantities of the insulin analog then cross the skin barrier through iontophoresis. The human insulin analog employed in practice of this invention is characterized by exhibiting a greater negative charge of at least one unit at neutral pH than that exhibited by human insulin.

By "insulin analogs" as used herein is meant a compound having a molecular structure similar to that of human insulin including the disulphide bridges between Cys(A7) and Cys(B7) and between Cys(A20) and Cys(B19) and an internal disulphide bridge between Cys(A6) and Cys(A11) and with insulin activity.

Desirably, the insulin analog medium is made the cathode in the electrical circuit through the skin and the pH of the insulin analog medium is in the range of pH 4 - 8.

The present invention also relates to novel human insulin analogs characterized by having improved properties for delivery by iontophoresis. These analogs have a reduced tendency to association and although they carry an increased net negative charge at neutral pH, analogs of this type have in iontophoretic in vitro experiments surprisingly been shown to have substantially enhanced transport through skin as compared to human insulin. The negative net charge is provided by substituting at least two of the amino acid residues in human insulin by a Glu or Asp residue.

The present insulin analogs can be characterized by having the following formula I:

```
A-Chain
                         S─────────────────────S
                         |      7               |
   Gly-Ile-Val-Glu-Y-Cys-Cys-Thr-Ser-Ile-Cys- W -
    1    2   3   4  5  6  |    8   9   10  11  12
                         S
                         |
B-Chain                  S
                         |
    Z - Z - Y -Y-His-Leu-Cys-Gly- X - X -Leu-Val
    1   2   3  4  5   6   7   8    9   10  11  12
```
                                                            (I)

```
A-Chain (contd.)
                              20
   Leu- W - Y -Leu-Glu- Y -Tyr-Cys- Z
    13   14  15  16  17  18  19  |   21
                              ┌──S
                              |
B-Chain (contd.)              S
                              |
   Glu-Ala-Leu- X -Leu- Z -Cys- Z -Glu- W -Gly-Phe-
    13   14  15  16  17  18  19  20  21  22  23  24
```

```
B-Chain (contd.)

   Phe- X - Z - X - W - W
    25   26  27  28  29  30
```

wherein the amino acid residue in at least one of the positions designated X or Z and furthermore the amino acid residue in at least one of the positions designated Y or W independently is substituted by Asp or Glu while any remaining X, Y, Z or W designates the amino acid residue occuring in human insulin in the pertinent position, the terminal ends of the B-chain optionally being extended by one or two amino acid residues which can be chosen independently among Asp and Glu.

One group of the above insulin analogs are such wherein in formula I one amino acid residue in a position designated X and furthermore the amino acid residue in at least one of the positions designated Y, Z or W independently is substituted by Asp or Glu while any remaining X, Y, Z or W designates the amino acid residue occuring in human insulin in the pertinent position, the terminal ends of the B-chain optionally being extended by one or two amino acid residues which can be chosen independently among Asp and Glu.

Another preferred group are such wherein in formula I two amino acid residues in positions designated X or Z and furthermore the amino acid residue in at least one of the positions designated Y or W independently is substituted by Asp or Glu while any remaining X, Y, Z or W designates the amino acid residue occuring in human insulin in the pertinent position, the terminal ends of the B-chain optionally being extended by one or two amino acid residues which can be chosen independently among Asp and Glu.

In the above insulin analogs one, two or three amino acid residues in a position designated W may independently be substituted with Asp or Glu. Also one, two or three of the amino acid residues in a position designated Y may independently be substituted by Asp or Glu.

If no W is substituted in formula I, or if the terminal ends of the B-chain have not been extended, then the total number of substitutions is preferably at least five. Preferred X positions for substitution are B9, B16, B26 and B28.

Examples of insulin analogs according to the present invention are:

```
Glu(B9),Glu(A12) human insulin,

Asp(B9),Glu(A12) human insulin,

Glu(B9),Asp(A12) human insulin,

Glu(B9),Glu(A14) human insulin,

Glu(B9),Glu(B22),Glu(B26) human insulin,

Asp(B9),Glu(B22),Glu(B26),Asp(A21) human insulin,

Glu(B3),Glu(B16),Asp(A12),Asp(A21) human insulin,

Glu(B16),Glu(B22) human insulin,

Asp(B16),Glu(B22) human insulin,

Glu(B16),Asp(A14) human insulin,

Glu(B16),Glu(B21),Asp(A14) human insulin,

Glu(B26),Glu(B29) human insulin,

Asp(B26),Glu(B30) human insulin,

Asp(B28),Glu(A12) human insulin,

Asp(B2),Glu(B16),Asp(A12),Asp(A21) human insulin,

Glu(B3),Glu(B9),Glu(B29) human insulin,

Glu(B9),Asp(B22),Glu(B31) human insulin,

Asp(B9),Asp(B16),Glu(B26),Glu(B28),Asp(A21) human insulin,

Asp(B16),Asp(B26),Glu(B31),Asp(B32) human insulin,

Asp(B22),Glu(B26) human insulin,

Glu(B9),Asp(B22),Glu(B31) human insulin and

Asp(B1),Asp(B4),Asp(B10),Asp(B16),Glu(B27) human insulin.
```

## DETAILED DESCRIPTION OF THE INVENTION

The novel insulin analogs according to the present invention can be prepared by altering the proinsulin gene through replacement of codon(s) at the appropriate site in the native human proinsulin gene by codon(s) encoding the desired amino acid residue substitute(s) or by synthesizing the whole DNA-sequence encoding the desired insulin analog. The gene encoding the desired insulin analog is then inserted into a suitable expression vector which when transferred to a suitable host organism, e.g. E. coli, Bacillus or yeast, generates the desired product. The expressed product is then isolated from the cells or the culture broth depending on whether the expressed product is secreted from the cells or not.

The novel insulin analogs may also be prepared by chemical synthesis by methods analogue to the method described by Märki et al. (Hoppe-Seyler's Z. Physiol.Chem., 360 (1979), 1619 - 1632). They may also be formed from separately in vitro prepared A- and B-chains containing the appropriate amino acid residue substitutions, whereupon the modified A-and B-chains are linked together by establishing disulphide bridges according to known methods (e.g. Chance et al., In: Rick, D.H., Gross, E. (Editors) Peptides: Synthesis - Structure - Function. Proceedings of the Seventh American Peptide Symposium, Illinois, pp. 721 - 728).

The insulin analogs may furthermore be prepared by a method analogue to the method described in EP patent application No. 0163529A, the disclosure of which is incorporated by reference hereinto. By such a method an insulin precursor of human insulin wherein Lys(B29) is connected to Gly(A21) by means of either a peptide bond or a peptide chain of varying length with correctly positioned disulphide bridges is expressed and secreted by yeast and then converted into human insulin by the so-called transpeptidation reaction.

Accordingly, the present insulin analogs may be prepared by inserting a DNA-sequence encoding a precursor of the insulin analog in question into a suitable yeast expression vehicle which when transferred to yeast is capable of expressing and secreting the precursor of the insulin analog in which Lys(B29) is connected to Gly(A21) by a peptide bond or a peptide chain with the formula II

$$-R_n-R^1- \qquad\qquad (II)$$

wherein R is a peptide chain with n amino acid residues, n is an integer from 0 to 33 and $R^1$ is Lys or Arg when culturing the transformed yeast strain in a suitable nutrient medium. The precursor is then recovered from the culture broth and reacted with an amino compound with the formula III

$$R^2-R^3-R^4-OR^5 \qquad\qquad (III)$$

wherein $R^2$ is Thr, Asp or Glu, $R^3$ and $R^4$ are each either Glu or Asp or a peptide bond, and $R^5$ is a carboxy protecting group (e.g. methyl or tert-butyl), using trypsin or trypsinlike enzyme as a catalyst in a mixture of water and organic solvents analogously as described in US patent specification No. 4,343,898 (the disclosure of which is incorporated by reference hereinto) whereupon the carboxy protecting group is removed and the insulin analog is isolated from the reaction mixture.

The insulin analogs may also be prepared by a method analogous to the method described in EP patent application No. 0195 691 the disclosure of which is incorporated by reference hereinto. By this method insulin precursors of the type having a bridge between the A- and B-chain consisting of a single pair of basic amino acid (Lys, Arg) are made in yeast and then converted into insulin by an enzymatic conversion.

Genes encoding the precursors of the insulin analog can be prepared by modification of genes encoding the corresponding human insulin precursors by site specific mutagenesis to insert or substitute with codons encoding the desired mutation. A DNA-sequence encoding the precursor of the insulin analog may also be made by enzymatic synthesis form oligonucleotides corresponding in whole or part to the insulin analog precursor gene.

DNA-sequences containing a gene with the desired mutation are then combined with a suitable promoter sequence, e.g. fragments coding for the TPI promoter (TPIp) (Alber, T. and Kawasaki, G., Nucleotide Sequence of the triose Phosphate Isomerase Gene of Saccharomyces cerevisiae. J.Mol. Applied Genet. **1** (1982), 419 - 434), a suitable leader sequence and possible transcription termination sequence, e.g. from TPI of <u>S</u>. <u>cerevisiae</u> (TPI$_T$). These fragments provide sequences to ensure a high rate of transcription for the precursor encoding gene and also provide a presequence which can effect the localization of precursor into the secretory pathway and its eventual excretion into the growth medium. The expression units are furthermore provided with a yeast origin of replication, for instance the 2μ origin, and a selectable marker, for instance LEU 2.

For the purpose of this invention we prepared the genes encoding the insulin analog precursor in question by ligation of oligonucleotides followed by insertion of the gene into a yeast expression plasmid as described by L. Thim et al., Proc.Natl.Acad.Sci. USA **83** (1986), 6766 - 6770, and J. Markussen et al., Protein Engineering **1** (1987), 215 - 223.

## Example 1

Production of Glu(B9), Glu(A12) human insulin

A synthetic gene for the precursor Glu(B9),B(1-29)-Ala-Ala-Lys-Glu(A21),A(1-21) was constructed from oligonucleotides by ligation. The oligonucleotides were synthesized on an automatic DNA synthesizer using phosphoramidite chemistry on a controlled pore glass support (Beaucage, S.L. and Caruthers, M.H. Tetrahydron Letters **22** (1981), 1859 - 1869). The synthetic precursor gene was composed as follows:

```
          B1 2    3   4   5   6   7   8   9  10  11  12  13  14  15  16  17
      K   R   F   V   N   Q   H   L   C   G   E   H   L   V   E   A   L   Y   L
```

```
AAAGATTCGTTAACCAACACTTGTGCGGTGAGCACTTGGTTGAAGCTTTGTACTTGG-
    AAGCAATTGGTTGTGAACACGCCACTGGTGAACCAACTTCGAAACATGAACC-
```

```
 18  19  20  21  22  23  24  25  26  27  28  29              A1  2   3   4
  V   C   G   E   R   G   F   F   Y   T   P   K   A   A   K   G   I   V   E
```

```
TTTGCGGTGAAAGAGGTTTCTTCTACACTCCTAAGGCTGCTAAGGGTATTGTCGAAC-
AAACGCCACTTTCTCCAAAGAAGATGTGAGGATTCCGACGATTCCCATAACAGCTTG-
```

```
  5   6   7   8   9  10  11  12  13  14  15  16  17  18  19  20  21
  Q   C   C   T   S   I   C   E   L   Y   Q   L   E   N   Y   C   N
```

```
AATGCTGTACCTCCATCTGCGAATTATACCAATTGGAAAACTACTGCAACT-
TTACGACATGGAGGTAGACGCTTAATATGGTTAACCTTTTGATGACGTTGA-
```

```
AGACGCAGCCCGCAGGCT
TCTGCGTCGGGCGTCCGAGATC
```

Letters and numbers above the DNA-sequence indicate the corresponding amino acid residues (using the one letter abbreviation) and their position, respectively, in the B- and in the A-chain. The sequence AAK (AlaAlaLys) is the bridge which connects the position B29 of the B-chain and position A1 of the A-chain.

By a method analogous to that described in WO 89/10937 a plasmid encoding the following sequence:

TPIp-MFα1-signal-leader(1-85)-precursor gene-TPI$_T$

where MFα1 is the <u>S</u>. <u>cerevisiae</u> MFα1 coding sequence (Kurjan, J. and Herskowitz, I., Cell **30** (1982) 933 - 943) and signal-leader(1-85) means that the sequence contains the first 85 amino acid residues of the MFα1 signal-leader sequence was obtained.

An <u>S</u>. <u>cerevisiae</u> (E2-7B XE11-36 a/α, Δtpi Δtpi, pep 4-3/pep 4-3) was grown on YPGaL (1% Bacto yeast extract, 2% Bacto peptone, 2% galactose, 1% lactate) to an optical density at 600 nm of 0.6.

100 ml of culture was harvested by centrifugation, washed with 10 ml of water, recentrifuged and resuspended in 10 ml of a solution containing 1.2 M sorbitol, 25 mM Na$_2$EDTA pH = 8.0, and 6.7 mg/ml dithiotreitol. The suspension was incubated at 30°C for 15 minutes, centrifuged and the cells resuspended in 10 ml of a solution containing 1.2 M sorbitol, 10 mM Na$_2$EDTA, 0.1 M sodium citrate, pH = 5.8, and 2 mg Novozym® 234. The suspension was incubated at 30°C for 30 minutes, the cells collected by centrifugation, washed in 10 ml of 1.2 M sorbitol and 10 ml of CAS (1.2 M sorbitol, 10 mM CaCl$_2$, 10 mM Tris-HCl (Tris = Tris(hydroxymethyl)aminomethan) pH = 7.5) and resuspended in 2 ml of CAS. For transformation 0.1 ml of CAS-resuspended cells were mixed with approximately 1 µg of the above described plasmid and left at room temperature for 15 minutes. 1 ml of (20% polyethylenglycol 4000, 10 mM CaCl$_2$, 10 mM Tris HCl, pH = 7.5) was added and the mixture left for further 30 minutes at room temperature. The mixture was centrifuged and the pellet resuspended in 0.1 ml of SOS (1.2 M sorbitol, 33% v/v YPD, 6.7 mM CaCl$_2$, 14 µg/ml leucine) and incubated at 30°C for 2 hours. The suspension was then centrifuged and the pellet resuspended in 0.5 ml of 1.2 M sorbitol. Then, 6 ml of top agar (the SC medium of Sherman et al., (Methods in Yeast Genetics, Cold Spring Harbor Laboratory, 1981) containing 1.2 M sorbitol plus 2.5% agar) at 52°C was added and the suspension poured on top of plates containing the same agar-solidified, sorbitol containing medium. Transformant colonies were picked after 3 days at 30°C, resisolated and used to start liquid cultures.

Transformant strains were grown on YPD medium (1% yeast extract, 2% peptone (from Difco laboratories), and 2% glucose) in a 1500 liter tank at 30°C. The expressed product was isolated from the culture broth.

Transpeptidation

L-threonine methyl ester acetate (38.6 g, 0.2 mole) was dissolved in dimethylformamide to give 100 ml of solution, 50 ml 76.5% v/v dimethylformamide in water was added and 10 g of crude Glu(B9),B(1-29)-Ala-Ala-Lys-Glu(A12),A(1-21) human insulin was dissolved in the mixture, which was thermostated at 12°C. Then 1 g of trypsin in 25 ml 0.05 M calcium acetate was added and after 24 hours at 12°C the mixture was added to 2 liters of acetone and the precipitated peptides were isolated by centrifugation and dried in vacuo. The Glu(B9),Glu(A21),B30Thr-OMe human insulin was purified on a preparative HPLC column with silica-C18 as column material.

The Glu(B9),Glu(A12),B30Thr-OMe human insulin was dispersed in water to 1% (w/v) and was dissolved by addition of 1 N sodium hydroxide to a pH value of 10.0. The pH value was kept constant at 10.0 for 24 hours at 25°C. The Glu(B9),Glu(A12) human insulin formed was precipitated by addition of sodium chloride to about 8% (w/v), sodium acetate trihydrate to about 1.4% (w/v), and zinc acetate dihydrate to about 0.01% (w/v) followed by addition of 1 N hydrochloric acid to pH 5.5. The precipitate of Glu(B9),Glu(A12) human insulin was isolated by centrifugation and purified by anion exchange chromotography and desalted by gel filtration.

**Example 2**

Production of Asp(B1),Asp(B4),Asp(B10),Asp(B16),Glu(B27) human insulin.

The title product was prepared by a method analogous to the method described in Example 1, using a synthetic precursor gene having the following composition:

```
     B1 2   3   4   5   6   7   8   9  10  11  12  13  14  15  16  17
  K  R  D   V   N   D   H   L   C   G   S   D   L   V   E   A   L   D   L
```

```
AAAGAGACGTTAACGATCACTTGTGCGGTTCCGACTTGGTTGAAGCTTTGGACTTGG-
     CTGCAATTGCTAGTGAACACGCCAAGGCTGAACCAACTTCGAAACCTGAACC-
```

```
  18 19 20 21 22 23 24 25 26 27 28 29               A1 2   3   4
  V  C  G  E  R  G  F  F  Y  E  P  K  A  A  K        G   I   V   E
```

```
TTTGCGGTGAAAGAGGTTTCTTCTACGAACCTAAGGCTGCTAAGGGTATTGTCGAAC-
AAACGCCACTTTCTCCAAAGAAGATGCTTGGATTCCGACGATTCCCATAACAGCTTG-
```

```
  5   6   7   8   9  10  11  12  13  14  15  16  17  18  19  20  21
  Q   C   C   T   S   I   C   S   L   Y   Q   L   E   N   Y   C   N
```

```
AATGCTGTACCTCCATCTGCTCCTTGTACCAATTGGAAAACTACTGCAACT-
TTACGACATGGAGGTAGACGAGGAACATGGTTAACCTTTTGATGACGTTGA-
```

```
AGACGCAGCCCGCAGGCT
TCTGCGTCGGGCGTCCGAGATC
```

Testing of Insulins by in vitro Iontophoresis.

Three different insulins, 1) human insulin, (1), 2) a monomeric insulin analog (2), with two extra negative charges in the molecule, Asp(B9),Glu(B27) human insulin, obtained as described in EP application No. 0 214 826, and 3) a monomeric insulin analog (3), with five extra negative charges in the molecule, Asp(B1),Asp(B4),Asp(B10),Asp(B16),-Glu(B27) human insulin, obtained as described in the present Example 2, were compared in vitro with respect to transdermal flux by iontophoresis.

The measurements were made in a cell as described by Glikfeld, P. et al., Pharmaceutical Research **5** (1988) 443 - 446. The anode and the cathode were positioned on the same side of a piece of hairless mouse skin which was stripped twenty times with adhesive tape. Ag/AgCl electrodes, used in order to minimize pH changes in the electrode chambers, were mounted 3 mm from the surface of the skin. A phosphate buffered (0.03 M, pH 7.4) insulin solution was placed in the cathode chamber and a similar buffer containing saline but without insulin was placed in the anode chamber. The cell was assembled with the skin separating the electrode compartments and the receptor compartment. The latter compartment was filled with phosphate buffer containing 0.3% bovine serum albumin and perfused with the same solution at a rate of 2 ml per hour. Iontophoresis was carried out at a constant current of 0.5 mA/cm$^2$ supplied to the electrodes from a commercial power supply for 6 hours. In the experiment the exposed area of the mouse skin was 0.55 cm$^2$ and the current applied was 0.28 mA. The 2 ml fractions of perfusion liquid collected every hour were analysed by insulin radio immunoassay.

Table 1 reports the results of the experiments. The concentration of insulin in the fractions collected during the experiment with human insulin (1) was below the detection limit. The transport per in vitro cell of the insulin analogs (2) and (3) which did pass the mouse skin is expressed relative to the concentration of the analog in question in the donor chamber, i.e. transport normalized by concentration.

Table 1

| Hours | Accumulated transport of test Compound X $10^3$ $(cm^3)$ | | |
|---|---|---|---|
| | (1) | (2) | (3) |
| 0 | 0 | 0 | 0 |
| 1 | 0 | 0.1 | 0.6 |
| 2 | 0 | 0.6 | 2.3 |
| 3 | 0 | 1.4 | 4.6 |
| 4 | 0 | 2.1 | 5.9 |
| 5 | 0 | 2.6 | 6.9 |
| 6 | 0 | 3.3 | 8.4 |

In summary, whereas no measurable flux of human insulin through the mouse skin could be detected during the 6 hours iontophoresis, a nearly constant flux was observed with the two monomeric analogs with the most negatively charged analog (3) exhibiting a flux 2-3 times higher than the flux of (2).

**Claims**

1.   Insulin analogs having the general formula I:

```
A-Chain
                                 S————————————————————S
                                 |     7                |
         Gly-Ile-Val-Glu-Y-Cys-Cys-Thr-Ser-Ile-Cys- W -
          1   2   3   4  5   6   |     8   9   10  11  12
                                 S
                                 |
         B-Chain                 S
                                 |
          Z - Z - Y -Y-His-Leu-Cys-Gly- X - X -Leu-Val
          1   2   3  4  5   6    7   8   9   10  11  12
```

```
A-Chain (contd.)
                                    20
         Leu- W - Y -Leu-Glu- Y -Tyr-Cys- Z
          13  14  15  16  17  18  19   |   21
                                    ┌——S
                                    |
         B-Chain (contd.)           S
                                    |
         Glu-Ala-Leu- X -Leu- Z -Cys- Z -Glu- W -Gly-Phe-
          13  14  15  16  17  18  19  20  21  22  23  24
```

```
B-Chain (contd.)

         Phe- X - Z - X - W - W
          25  26  27  28  29  30
```

(I)

wherein the amino acid residue in at least one of the positions designated X or Z and furthermore the amino acid residue in at least one of the positions designated Y or W independently is substituted by Asp or Glu while any remaining X, Y, Z or W designates the amino acid residue occuring in human insulin in the pertinent position, the terminal ends of the B-chain optionally being extended by one or two amino acid residues which can be chosen independently among Asp and Glu with the proviso that if no amino acid residue in a position designated W is substituted, or if the terminal ends of the B-chain have not been extended, then the total number of substitutions is at least five.

2. Insulin analogs according to Claim 1 wherein one amino acid residue in a position designated X and furthermore the amino acid residue in at least one of the positions designated Y, Z or W independently is substituted by Asp or Glu while any remaining X, Y, Z or W designates the amino acid residue occuring in human insulin in the pertinent position, the terminal ends of the B-chain optionally being extended by one or two amino acid residues which can be chosen independently among Asp and Glu.

3. Insulin analogs according to Claim 2 wherein two amino acid residues in positions designated X or Z and furthermore the amino acid residue in at least one of the positions Y or W independently is substituted by Asp or Glu while any remaining X, Y, Z or W designates the amino acid residue occuring in human insulin in the pertinent position, the terminal ends of the B-chain optionally being extended by one or two amino acid residues which can be chosen independently among Asp and Glu.

4. Insulin analogs according to any of the predecing claims wherein the total number of substitutions is at least five.

5. Insulin analogs according to any of the preceding claims wherein one amino acid residue in a position designated W is substituted by Asp or Glu.

6. Insulin analogs according to any of the claims 1 -4 wherein two amino acid residues in positions designated W independently are substituted by Asp or Glu.

7. Insulin analogs according to any of the claims 1 -4 wherein three amino acid residues in positions designated W independently are substituted by Asp or Glu.

8. Insulin analogs according to any of the claims 1 -4 wherein one amino acid residue in a position designated Y is substitued by Asp or Glu.

9. Insulin analogs according to any of the claims 1 -4 wherein two amino acid residues in positions designated Y independently are substituted by Asp or Glu.

10. Insulin analogs according to any of the claims 1 -4 wherein three amino acid residues in positions designated Y independently are substituted by Asp or Glu.

11. A pharmaceutical composition comprising an insulin analog according to any of the preceding claims.

12. The use of an insulin analog according to any of the claims 1 - 10 for the manufacture of a pharmaceutical composition for the treatment of diabetes.


**Patentansprüche**

1. Insulinanaloge mit der allgemeinen Formel I:

A-Kette

```
                                      S————————————S
                                      |      7       |
        Gly-Ile-Val-Glu-Y-Cys-Cys-Thr-Ser-Ile-Cys- W -
         1   2   3   4  5  6   7    8   9   10  11  12
                                      |
                                      S
B-Kette                               |
                                      S
                                      |
         Z - Z - Y -Y-His-Leu-Cys-Gly- X - X -Leu-Val
         1   2   3  4  5   6   7   8    9  10  11  12
```

A-Kette (Forts.)

```
                                   20
        Leu- W - Y -Leu-Glu- Y -Tyr-Cys- Z
         13  14  15  16  17  18  19   |   21
                                    r—S
                                    |
B-Kette (Forts.)                    S
                                    |
        Glu-Ala-Leu- X -Leu- Z -Cys- Z -Glu- W -Gly-Phe-
         13  14  15  16  17  18  19  20  21  22  23  24
```

B-Kette (Forts.)

```
        Phe- X - Z - X - W - W
         25  26  27  28  29  30
```

(I)

in der der Aminosäurerest in wenigstens einer der mit X oder Z bezeichneten Positionen und außerdem der Aminosäurerest in wenigstens einer der mit Y oder W bezeichneten Positionen unabhängig substituiert ist durch Asp oder Glu, während jedes übrigbleibende X, Y, Z oder W den Aminosäurerest bezeichnet, der in der entsprechenden Position in Humaninsulin auftritt, wobei die terminalen Enden der B-Kette fakultativ um ein oder zwei Aminosäurereste verlängert sind, die unabhängig aus Asp und Glu ausgewählt werden können, mit der Maßgabe, daß, wenn kein Aminosäurerest in einer mit W bezeichneten Position substituiert ist oder wenn die terminalen Enden der B-Kette nicht verlängert worden sind, dann die Anzahl der Substitutionen insgesamt wenigstens fünf beträgt.

2. Insulinanaloge nach Anspruch 1, wobei ein Aminosäurerest in einer mit X bezeichneten Position und außerdem der Aminosäurerest in wenigstens einer der mit Y, Z oder W bezeichneten Positionen unabhängig durch Asp oder Glu substituiert ist, während jedes übrigbleibende X, Y, Z oder W den Aminosäurerest bezeichnet, der in der entsprechenden Position in Humaninsulin auftritt, wobei die terminalen Enden der B-Kette fakultativ um ein oder zwei Aminosäurereste verlängert sind, die unabhängig aus Asp und Glu ausgewählt werden können.

3. Insulinanaloge nach Anspruch 2, wobei zwei Aminosäurereste in mit X oder Z bezeichneten Positionen und außerdem der Aminosäurerest in wenigstens einer der Positionen Y oder W unabhängig durch Asp oder Glu substituiert ist, während jedes übrigbleibende X, Y, Z oder W den Aminosäurerest bezeichnet, der in der entsprechenden Position in Humaninsulin auftritt, wobei die terminalen Enden der B-Kette fakultativ um ein oder zwei Aminosäurereste verlängert sind, die unabhängig aus Asp und Glu ausgewählt werden können.

4. Insulinanaloge nach einem der vorangehenden Ansprüche, wobei die Anzahl der Substitutionen insgesamt wenigstens fünf beträgt.

5. Insulinanaloge nach einem der vorangehenden Ansprüche, wobei ein Aminosäurerest in einer mit W bezeichneten Position durch Asp oder Glu substituiert ist.

6. Insulinanaloge nach einem der Ansprüche 1-4, wobei zwei Aminosäurereste in mit W bezeichneten Positionen unabhängig durch Asp oder Glu substituiert sind.

7. Insulinanaloge nach einem der Ansprüche 1-4, wobei drei Aminosäurereste in mit W bezeichneten Positionen unabhängig durch Asp und Glu substituiert sind.

8. Insulinanaloge nach einem der Ansprüche 1-4, wobei ein Aminosäurerest in einer mit Y bezeichneten Position durch Asp oder Glu substituiert ist.

9. Insulinanaloge nach einem der Ansprüche 1-4, wobei zwei Aminosäurereste in mit Y bezeichneten Positionen unabhängig durch Asp oder Glu substituiert sind.

10. Insulinanaloge nach einem der Ansprüche 1-4, wobei drei Aminosäurereste in mit Y bezeichneten Positionen unabhängig durch Asp oder Glu substituiert sind.

11. Eine pharmazeutische Zusammensetzung, die ein Insulinanalog nach einem der vorangehenden Ansprüche umfaßt.

12. Die Verwendung eines Insulinanaloge nach einem der Ansprüche 1-10 zur Herstellung einer pharmazeutischen Zusammensetzung für die Behandlung von Diabetes.

**Revendications**

1. Analogues d'insuline ayant la formule générale I :

**Chaîne A**

```
                                    S――――――――――――――S
                                    |      7       |
Gly-Ile-Val-Glu-Y-Cys-Cys-Thr-Ser-Ile-Cys- W -
  1   2   3   4  5  6   |   8   9   10  11  12
                        S
                        |
```

**Chaîne B**

```
                        S
                        |
  Z - Z - Y -Y-His-Leu-Cys-Gly- X - X -Leu-Val
  1   2   3  4   5   6   7   8   9   10  11  12
```

**Chaîne A (suite)**

```
                              20
Leu- W - Y -Leu-Glu- Y -Tyr-Cys- Z
 13  14  15  16  17  18  19   |   21
                              ――S
                              |
                              S
                              |
```

**Chaîne B (suite)**

```
Glu-Ala-Leu- X -Leu- Z -Cys- Z -Glu- W -Gly-Phe-
 13  14  15  16  17  18  19  20  21  22  23  24
```

**Chaîne B (suite)**

```
Phe- X - Z - X - W - W
 25  26  27  28  29  30
```

**(I)**

dans lesquels le résidu d'acide aminé dans au moins l'une des positions désignées X ou Z et de plus le résidu d'acide aminé dans au moins l'une des positions désignées Y ou W indépendamment sont substitués par Asp ou Glu tandis que tout X, Y, Z ou W restant désigne le résidu d'acide aminé produit dans l'insuline humaine dans la position pertinente, les extrémités terminales de la chaîne B étant facultativement prolongées d'un ou de deux résidus d'acide aminé pouvant être choisis indépendamment parmi Asp et Glu à condition que si aucun résidu d'acide aminé dans une position désignée W n'est substitué, ou si les extrémités terminales de la chaîne B n'ont pas été prolongées, le nombre total de substitutions sera alors au moins égal à cinq.

2. Analogues d'insuline selon la revendication 1, dans lesquels un résidu d'acide aminé dans une position désignée X et de plus le résidu d'acide aminé dans au moins une des positions désignées Y, Z ou W indépendamment, sont substitués par Asp ou Glu tandis que tout X, Y, Z ou W restant désigne le résidu d'acide aminé se produisant dans l'insuline humaine dans la position pertinente, les extrémités terminales de la chaîne B étant facultativement prolongées d'un ou de deux résidus d'acide aminé pouvant être choisis indépendamment parmi Asp et Glu.

3. Analogues d'insuline selon la revendication 2, dans lesquels deux résidus d'acide aminé dans des positions désignées X ou Z et de plus le résidu d'acide aminé dans au moins l'une des positions Y ou W indépendamment, sont substitués par Asp ou Glu tandis que tout X, Y, Z ou W restant désigne le résidu d'acide aminé produit dans l'insuline humaine dans la position pertinente, les extrémités terminales de la chaîne B étant facultativement prolongées d'un ou de deux résidus d'acide aminé pouvant être choisis indépendamment parmi Asp et Glu.

4. Analogues d'insuline selon l'une quelconque des revendications précédentes, dans lesquels le nombre total de

EP 0 568 594 B1

substitutions est au moins égal à cinq.

5. Analogues d'insuline selon l'une quelconque des revendications précédentes, dans lesquels un résidu d'acide aminé dans une position désignée W est substitué par Asp ou Glu.

6. Analogues d'insuline selon l'une quelconque des revendications 1 - 4, dans lesquels deux résidus d'acide aminé dans des positions désignées W indépendamment sont substitués par Asp ou Glu.

7. Analogues d'insuline selon l'une quelconque des revendications 1 - 4, dans lesquels trois résidus d'acide aminé dans des positions désignées W indépendamment sont substitués par Asp ou Glu.

8. Analogues d'insuline selon l'une quelconque des revendications 1 - 4, dans lesquels un résidu d'acide aminé dans une position désignée Y est substitué par Asp ou Glu.

9. Analogues d'insuline selon l'une quelconque des revendications 1 - 4, dans lesquels deux résidus d'acide aminé dans des positions désignées Y indépendamment sont substitués par Asp ou Glu.

10. Analogues d'insuline selon l'une quelconque des revendications 1 - 4, dans lesquels trois résidus d'acide aminé dans des positions désignées Y indépendamment sont substitués par Asp ou Glu.

11. Composition pharmaceutique aomprenant un analogue d'insuline selon l'une quelconque des revendications précédentes.

12. Utilisation d'un analogue d'insuline selon l'une quelconque des revendications 1 - 10 pour la fabrication d'une composition pharmaceutique destinée au traitement du diabète.

15